# EUROPEAN PATENT SPECIFICATION

(11) **EP 2 424 467 B1**
(45) Date of publication and mention of the grant of the patent: **23.04.2014**
(21) Application number: 09787720.3
(22) Date of filing: 30.04.2009
(51) Int. Cl.: A61F 2/16

(54) **INTRAOCULAR LENS WITH SYSTEM OF LASER ADJUSTABLE HAPTICS**
INTRAOKULARLINSE MIT SYSTEM AUS LASERVERSTELLBARER HAPTIK
LENTILLE INTRAOCULAIRE AVEC SYSTÈME D'HAPTIQUES AJUSTABLES PAR LASER

(43) Date of publication of application: 07.03.2012
(73) Proprietor: Promacon Italia Oftalmologia S.R.L., 95030 Tremestieri Etneo CT (IT)
(72) Inventor: GRASSO, Santi, I-95030 Tremestieri Etneo CT (IT); ORECCHIO, Fabio, I-95030 Tremestieri Etneo CT (IT)
(74) Representative: Romano, Giuseppe
(86) International application number: PCT/IT2009/000196
(87) International publication number: WO 2010/125596

(56) References cited:
- EP-A- 1 743 601
- WO-A-01/50984
- WO-A-2008/107882
- GB-A- 2 181 355
- US-A- 5 843 188
- US-A1- 2007 135 915
- US-A1- 2009 005 865
- US-B1- 6 443 984

## Description

### Technical Field

The present invention is related with the sector of ophthalmic implants, more specifically with the intraocular lenses which are employed in cataract surgery, in the clear crystalline lens surgery or in phakic patients for correction of refractive defects. Among them it is included in the field of Intraocular Lenses which power can be adjusted after the surgery.

### Background of the invention

Frequently, in the ophthalmic surgery, it is necessary to implant lenses inside the eye to achieve a satisfactory refractive state. This is more usual in the cataract surgery in which the natural crystalline lens is opaque and it is extracted and substituted by an artificial Intraocular Lens. The development of advanced Formulas for the calculation of the Intraocular Lens power and of more exact tools to measure the dimensions of the eye and its curvatures has allowed a high precision to achieve the desired postoperative refraction after the crystalline lens surgery. There are cases however where still after carrying out a technically perfect surgery the reached outcome is a poor not corrected vision due to a refractive result that was not targeted.

This is less common in eyes with normal morphology, but becomes frequent in eyes outside of the average dimensions; either big, small, with disproportionate depths of anterior and posterior segments, or with atypical corneal curvatures as happens in patients who previously were done corneal refractive surgery. Also in eyes that have silicone oil like substitute of the vitreous, among others. In these cases the formulas do not provide good results or measurements of the ocular globe lack of accuracy.

The incorrect power of Intraocular lenses is the number one cause of surgical removal and secondary reoperation. According to some statistics between 70% and 90% of the patients operated of cataract will be inside ±1.0 Diopter of error of the wanted postoperative refraction. Even in the hands of the most qualified professionals large refractive surprises happen. Their incidence is very variable, from 7.1% to 20% according to the reported by different studies.

In cases where the refractive surprise could not be avoided, there are different approaches to correct this trouble. The use of Eyeglasses or Contact Lenses creates many inconveniences and is not always possible, so surgery is frequently required. The exchange of Intraocular Lenses, and the secondary implantation of a second Intraocular Lens (Piggy back) can have serious potential complications, as well as LASIK (Laser in situ Keratomileusis) and .other corneal surgical procedures. Therefore, an alternative that can provide a permanent solution for the refractive surprise in a safe non invasive way would be an important tool in the hands of the ophthalmologists to solve this undesirable and frequent event seen daily in the practice of the eye surgeons.

### State of the art

Multiple Intraocular Lenses have been designed to have adjustable power after the surgery to correct refractive surprises or to adjust the postoperative refraction. In the patents and literature reviewed it has not been found coincidence with the design or the principles of the Adjustable Intraocular Lens described in this document.

The North American patents, US 7,210,783 B2 (Jethmalani et al) and 0260311 A1(Jethmalani et al 2007), show an Intraocular lens adjustable with the light or heat, made of photosensitive polymer embedded inside a polymer matrix, it can be adjusted in a non invasive way by means of a digital system of application of light that would change the power of the lens after the wound of the cataract surgery has been stabilized. This approach is very interesting and it can allow correcting not only the spherical defect, but also the astigmatism and other aberrations. But the new materials require extensive studies of their properties and biocompatibility before being accepted. Such Intraocular lenses if prove safety could be very expensive and the ophthalmologist would need to have a specific Laser machine for the adjustment.

The US2003/0135271 A1 (Bandhauer) describe an adjustable Intraocular Lens that has one or several frangible structures among the haptics and the optic that can be cut to adjust the position of the Intraocular Lens.

The US 6,443,984 B1 (Jahn et al) protects an adjustable intraocular Lens with mechanism based on screws or Cylinders and pistons in the optics and the haptics to displace the optic of the lens. These have the inconvenience that a second surgery is necessary to adjust the refraction, there is not any significant advantage therefore in this.

(Anello et al 1999) US 5,984,692 propose an Adjustable Intraocular Lens in which the optic can be rotated in the postoperative period by manual methods, Laser or micro motors to adjust the focus or astigmatic correction.

In the US 4,950,289 (Krasner 1990) an Adjustable Lens is described for small incisions with a deformable soft optic formed by a necklace and a bag in which fluid can be injected to vary the power of the lens.

The US 2007/0299487 A1 (Shadduck 2007) is about an adaptive Lens composed by moveable structures that include a photosensitive polymer that finely could change the bend of the surface of the lens.

In the US 6960231 B2 (Son 2005) the Lens consists of a non optic component with ring shape and an optic area that is placed in the first one where it can be adjusted mechanically. In the US 7,068,439 B2 (Esch et al) the lens consists of multiple cells full with fluid which quantity can be modified by an external source of energy to vary in this way the bend of the lens. In the US 2004 / 0106993 (Portney) a System of lenses is described in which a second lens can be inserted if necessary in present fissures in the primary implant.

The WO 2003/084441 20031016 shows an adjustable Lens with system of two optics united by expansible material that allows the central placement and axial displacement of the optic, and in the US 6730123 B1 (Klopotek 2004) an adjustable Lens is described by means of system of fluid and valves.

Other inventions have been patented in this field like the US 2725575 (O'Donnell, 1998); 5800533 Eggleston et al 1998; 0148022 A1 Eggleston 2004; US 2005/0182489 A1 (Gholam et al); 0133228 A1 (Sarver 2002). These either because of the complexity of their production or possible use have not been introduced in the market.

A lens according to the preamble of claim 1 is known from the document WO-A-2008/107882.

### Summary of the invention

Object of the present invention is to obviate the drawbacks mentioned above with reference to the known art, by providing a device as substantially described in claim 1.

Secondary features of the present invention are instead defined in the respective dependent claims. ,

The present invention, by overcoming the mentioned problems of the known art, entails several evident advantages.

Based on the problem previously described the author designed the Intraocular Lens with system of Laser adjustable haptics that would allow an easy non invasive adjustment of the refraction in cases of refractive surprise after surgery of the crystalline lens or another ocular surgery. The external appearance can be very similar to the current intraocular lenses, so it should be very familiar and accepted by the ophthalmologic community. The materials to manufacture it can be the same ones used in the current lenses and the industrial process of production can be carried out by the current art and existent technology.

The invention described here consists of an Intraocular Lens that can be adjusted inside the eye after cataract surgery, Refractive Surgery of the clear crystalline lens or any other ocular surgery; causing a movement directed backward or to the front of the Optic of the lens. This result is achieved in a non invasive way that is familiar and available for most of the ophthalmologists like the application of Laser. (YAG Laser, Argon laser or any other safe for ophthalmic use depending on the materials employed in the lens).

Other advantages, features and the operation modes of the present invention will be made apparent from the following detailed description of preferred embodiments thereof, given by way of example and not for limitative purposes.

### Brief description of the drawings

Reference will be made to the figures of the annexed drawings, wherein:
- Figures 1a to 1c show a first embodiment of an intraocular lenses 1 according to the present invention, having haptic fragments comprising arched elastic members;
- Figures 2 and 3 are respectively a schematic anterior-posterior view and a side view of a haptic fragment in its initial state when it has not been treated with laser and maintains a straight position;
- Figures 4 and 5 are respectively a schematic anterior-posterior view and a side view of a haptic fragment which is treated with one laser shot on the arched element, which has the bend down;
- Figures 6 and 7 are respectively a schematic anterior-posterior view and a side view of a haptic fragment which is treated with two laser shot on the arched element, which has the bend down;
- Figures 8 and 9 are respectively a schematic anterior-posterior view and a side view of a haptic fragment which is treated with three laser shot on the arched element, which has the bend down;
- Figure 10 shows an anterior-posterior view of one of the preferred designs of Intraocular Lens with system of laser adjustable haptics;
- Figure 11 shows a lateral view of the Adjustable Intraocular lens of figure 10;
- Figure 12 is a three-dimensional view of the Adjustable Intraocular Lens of the Figures 10 and 11;
- Figure 13 shows a cross section of the Anterior Segment of eye implanted with Adjustable Intraocular Lens of the Figures 10, 11 and 12, which has not been adjusted with Laser, so the haptics are straight;
- Figure 14 shows a cross section of the Anterior Segment of eye implanted with Adjustable Intraocular Lens of the Figure 13 which has been adjusted with Laser to correct hyperopic defect;
- Figure 15 shows a cross section of the Anterior Segment eye implanted with Adjustable Intraocular Lens of the Figure 13 which has been adjusted with Laser to correct myopic defect;
- Figure 16 shows the antero-posterior view of a second embodiment of intraocular Lens with system of adjustable haptics according to the present invention;
- Figure 17 shows a side view of the intraocular Lens with system of adjustable haptics of the Figure 16 which has not been adjusted with Laser, so the haptics are straight;
- Figure 18 shows a side view of the intraocular Lens of Figure 16 when treated with Laser to correct hyperopic defect;
- Figure 19 shows the side view of the intraocular Lens of Figure 16 when treated with Laser to correct myopic defect;
- Figure 20 shows a third embodiment of Intraocular Lens with adjustable haptics according to the present invention; and
- Figure 21 shows a fourth embodiment of Intraocular Lens with adjustable haptics according to the present invention.

### Detailed description of the invention

The present invention will hereinafter be described making reference to the above-indicated figures.

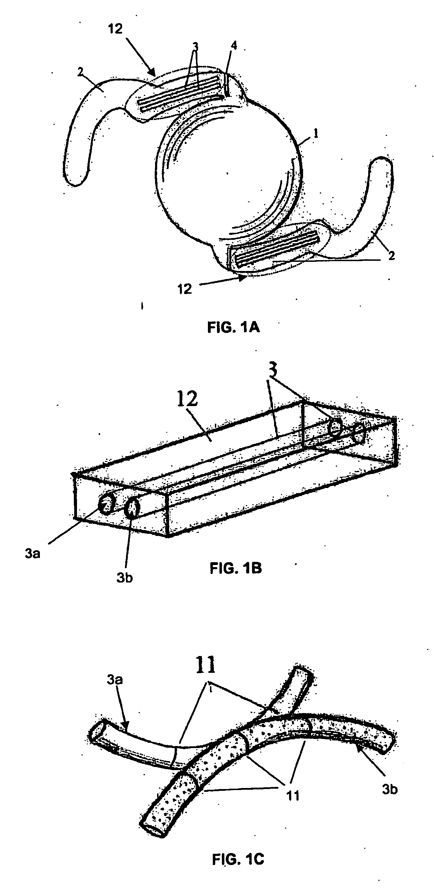

Referring initially to Figures 1a to 1c, they show an intraocular lens 1 and its components, according to the present invention.

The intraocular lens consists of an optic 1 of 5.0 mm, 5.25 mm, 5.5 mm, 5.75 mm, 6.0 mm or any other wanted diameter, its shape and materials can be the same ones used in the existent Intraocular lenses in the current state of the art of proven quality and safety (Example: Hydrophilic or hydrophobic Acrylic, silicone, or other). Preferably foldable, it can have associated characteristics as multifocality, asphericity, filter for the blue light or UV, toric, square edges, etc.

The lens according to the present invention comprises a mechanism in the supporting arms, also named haptics 2, that allows an adjustment of the refractive sphere in a patient who has been implanted the lens.

The lens 1, has a pair of haptics 2, as supporting arms. The haptics 2 can be made of the same piece of the optic 1. A haptic fragment is indicated with the numeral 12, as seen in Figure 1b. Each of the fragments 12 comprises one or more pair of arched elastic members 3 substantially placed along a longitudinal direction of the haptic.

This haptic fragment 12 correspond to the area of the haptic 2 circled in discontinuous line in Figure 1a. The positions that the arched members 3 have inside the haptic are illustrated in Figure 1b. They have respective bends in opposite direction anteriorly or posteriorly, as also illustrated in Figure 1 c. Because of the contrary forces of the arched elastic members 3, one annuls the bend of the other and the haptic 2 remain substantially straight.

The elastic arched members 3 are in the form of fine cylinders or bars or differently shaped elongated members which longitude and thickness depend on the external design and size of the haptic.

These arched members 3 are preferably made of a flexible material of suitable elasticity, force, shape memorizing property and biocompatibility. Better of well-known material with proven safe intraocular use like Polypropylene, Polimide, Polimetilmetacrilate (PMMA), or another. The material should also have the property to weaken or to lose its elastic force when being shot with laser. These members have the shape of arches 3 with a certain curvature radius. The arches 3 can be bended at least to a straight position by applying a certain force and when moving away this force the elastic properties and memory of the material should make them return to the initial arched shape. The arch 3 should exert enough force to bend the complete haptic in which it is included.

These elastic arched members 3 are introduced during the manufacture process of the lens inside the haptic 2 of foldable material as acrylic, silicone, hydrogel or another. For each arched member introduced with the bend directed forward another should be included in its side with the bend of the arch in the opposite direction. The arched elastic cylinders or bars 3 can be shaped, for example at least in one of their distal end, so to avoid the arch to rotate and take a not wanted position inside the haptic. As an example they could have at their distal end a small termination in form of "T" or a flat end.

The number of curved elements 3 to be introduced in each haptic can be varied. Only two, would require a smaller haptic, an easier process of manufacture and surgically it would be a friendlier lens. Four or more flexible arched cylinders in each haptic can provide more force to change the position of the lens, and bigger displacement range, allowing therefore adjusting the postoperative refraction widely and finely.

The arched elastic members 3 of are placed side by side or.

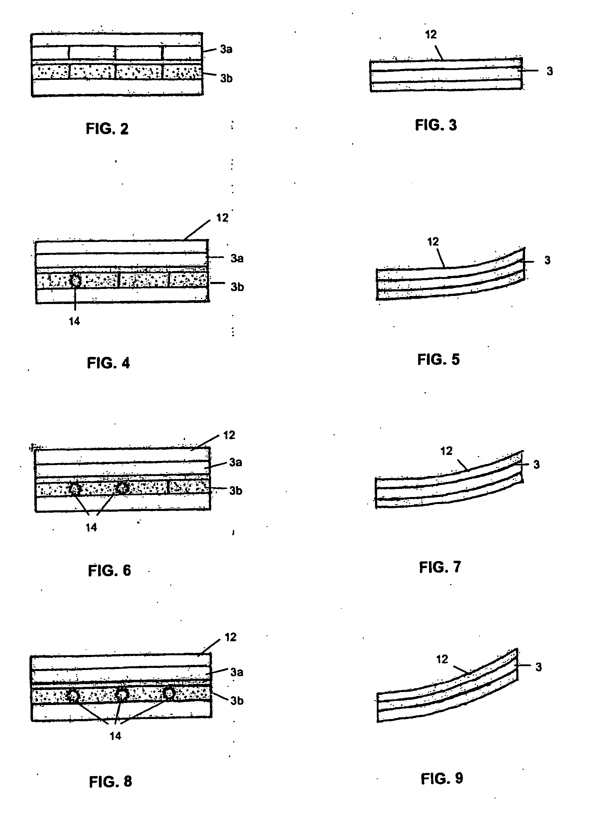

Making reference to figures 2 to 9, they illustrate how the postoperative refraction of a lens according to the present invention can be adjusted.

When the elastic arches 3 are positioned inside the haptic 2 with their curves in the opposite position, very near one to the other, their contrary forces compensate the bends of each other and the haptic remains substantially straight, as depicted in figures 2 and 3.

If one of the arched elements 3 loses its force this balance breaks and the other one bends taking its primary curved shape, also bending by means of the force exercised by it, the complete haptic and moving the optic of the lens 1 in the direction of the bend.

In order to cause an elastic arch 3 to loose its elastic property, it can be shot with a Laser. It is not necessary to break it completely, only to weaken it making it lose its elastic property and force in one or several points.

Marks 11 are advantageously provided on each arched member 3. This allows the operator to exactly identify the shooting point.

The laser shot can be placed in one of the marks 11 provided on the elastic arch to know the degree of bending of the haptic and displacement of the optic that will take place with the laser shot in that certain place, which will mean a certain change of the patient's refraction.

If the shot of the laser 14 is done in the external third of the arch, the other two thirds can still bend and partially can oppose to the force from the intact arched cylinder at its side, only allowing a partial movement of the optic. This situation is illustrated in figures 4 and 5.

Figure 5 shows the side view of the haptic fragment 12 schematically shown in figure 4 which received laser shot in the arched cylinder bended down 3b with that it weakens and the arched cylinder bended up 3a acquires a positive correlation of force when having less resistance from the treated cylinder 3b, bending lightly up and bowing also the haptic of flexible material.

If a second shot of the laser is placed on the center of the cylinder, the weakness is bigger and the other arch bends more (FIGS. 6, 7) causing a bigger displacement of the optic.

Figure 6 shows the anterior-posterior view of a haptic fragment 12 which is treated with two laser shots 14 on the arched element 3b, which has its bend down. The shot with laser is placed in the marks 11 that the elastic arch has to predict the degree of bending of the haptic and displacement of the optic that will take place with the shot with laser in that certain place, that will mean an increase in the change of the patient's refraction.

Figure 7 shows the side view of the haptic fragment 12 schematically shown in figure 6 which received a second laser shot in the arched element bended down 3b with that it weakens and the arched cylinder bended up 3a suffers a smaller resistance bending up more and bowing also the haptic of flexible material.

One or several more shots with the laser would finish any resistance against the flexion and the intact arch will bend to its biggest possibility causing the maximum movement of the optic 1.

As an example, Figure 8 shows the schematic anterior-posterior view of a haptic fragment 12 which is treated with three laser shots 14 on the arched element 3b, which has its bend down. The shot with laser is placed in the marks 11 that the elastic arch has to predict the grade of bowing of the haptic and displacement of the optics that will take place with the shot with laser in that certain place, that will mean an increase in the change of the patient's refraction.

Figure 9 shows the schematic side view of the haptic fragment 12 shown in figure 8 which received a third laser shot in the arched cylinder 3 bended down 3b, with that it weakens even more and the arched cylinder up 3a bends up more increasing the bending of the haptic.

In an ideal way the adjustment should be made in steps of 0.25 Diopters or 0.50 Diopters, although it can also be made in larger steps. While more bending can be achieved in the haptic, a bigger displacement of the optic will be achieved in the antero-posterior axis of the eye and the lens will be able to correct bigger refractive defects. The radius of curvature, the thickness and the longitude of the elastic arches 3 to achieve the wanted displacement will depend on the properties of the materials used in the elastic arched elements 3 and in the rest of the intraocular Lens which design, hardness and resistance to the flexion will determine the force required to cause this movement. Arched elements with the force, length and correct curvature radius should be used to achieve the progressive displacements that allow the fine adjustment of the postoperative refraction.

That is to say that the balance of forces between the arched elements 3 can be broken, this would stop to maintain the haptics straight, curving them forward or backward, and displacing the optic of the lens 1 moving it away or bringing it near to the retina as it is required in each patient to correct the existent refractive defect.

This postoperative adjustment can be made from days to months after the surgery, or even years, but preferably in a term of weeks when the postoperative refraction has already been stabilized and still there is not fibrosis of the capsular sack of the crystalline lens.

The arched components 3 can preferably have different colors or marks according to the direction of the arch. This indicates the Ophthalmologist to what elastic arch to shoot to treat myopia or hyperopia. (Red and Blue e.g.). The arched elements can also have marks 11 that point out where to shoot with the laser to produce a certain quantity of displacement and a proportional refractive change.

If the Lens is provided with four arched members 3 in each haptic instead of two, the quantity of displacement can also be regulated by treating only one, or two cylinders in each haptic. This can allow a finer adjustment of the postoperative position of the lens and the refraction.

According to the revised bibliography, 1 mm of displacement of the Intraocular Lens in the Anterior-posterior Axis of the eye generates a significant change of the reached refraction. The magnitude of the change depends on the size of the eye:
Small eyes (Average 21.44 mm): 1mm of displacement means 1.9 Diopters.
Normal eyes (Average 23.28 mm): 1mm of displacement means 1.4 Diopters.
Big eyes (Average. 27.09 mm): 1 mm of displacement means 0.6 Diopters.

This means that in the eyes of average size a displacement of 1mm forward or backward of the Intraocular Lens, causes a change in the refraction of 1.4 Diopters; a displacement of 0.7mm induces a difference of 1.0 Diopter; 0.35mm means 0.5 Diopters and 0.17mm only affects the refraction in 0.25 Diopters. This effect is magnified in small eyes and it diminishes in the large eyes. Therefore the Lenses of higher powers that are used in the smaller eyes can be manufactured with arched elements that generate less movement than those that are implanted in Intraocular lenses of lower power that should make a larger excursion to produce the same refractive change.

Ideally the Intraocular Lens with system of laser adjustable haptic should be able to make a large displacement that allow to correct defects that include the great majority of the refractive surprises (Example, a displacement to correct from +3.00 Diopters to -3.00 Diopters) and that the fine tuning of its adjustment be in small steps of 0.25D or 0.50D. If technically difficult to manufacture a Lens that achieves a range of displacement so wide as to correct 3.00 positive or negative Diopters, an amplitude of 1.50 Diopters would include also a great majority of operated patients, making of these Intraocular lenses an important tool to achieve an excellent visual result in most of the patients operated of cataract or another ocular condition.

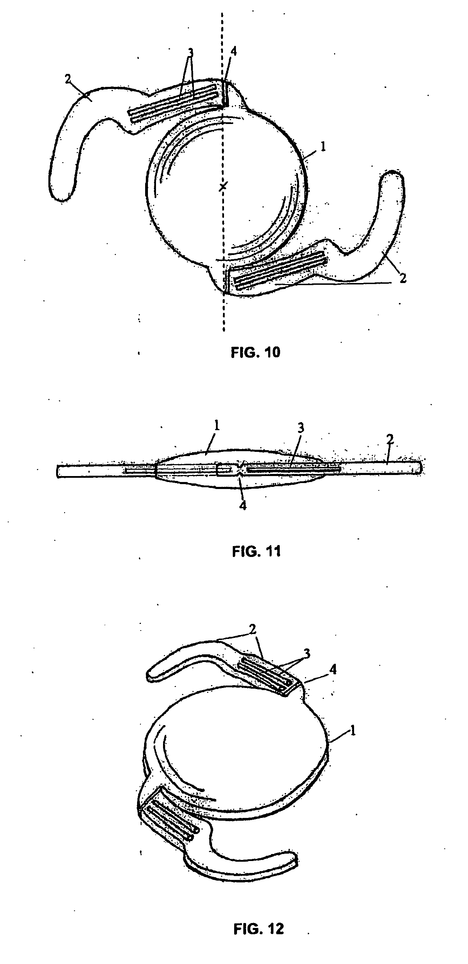

Figure 10 The optic area 1 can be similar to any other Intraocular Lens in the current state of the art.

Next Figures 10, 11 and 12, show a preferred embodiment of intraocular lens according to the present invention.

A lens 1 is made of a single piece of foldable material as acrylic, silicone or other. The haptics 2 of the lens 1 are of foldable material of the same piece of the optic. They have the shape of a double arch, the first one is more straight and nearer to the optic forming a narrow angle with it. The angle opens up in the distal half of the haptic reaching the Lens an external diameter of 13 mm or another wanted. The shape of the haptics allows that when the lens is placed in the eye, inside the capsular sack, its external diameter decreases to approximately 10 mm, the most central portion in the haptics where the elastic arched components 3 are placed, get closer to,the edge of the optics and they can be treated with Laser through mydriatic pupil of 7 or 8mm.

The Lens has hinges or articulations 4 in the junction of the haptics with the optic to facilitate the flexion at this level. The hinges are aligned one with the contralateral following an imaginary line that crosses the optic through the center, so that when the haptics bend they do not tilt the optic 2.

A lateral view of the lens 1 is shown in Figure 11, from which can be appreciated the profile of the lens. The optic's profile, in this case illustrated as a biconvex profile, can be any other wanted (Plane-convex, concave-convex, biconcave, aspheric, bifocal, multifocal or other). The thinning of the haptic 2 is illustrated in the articulation or hinge 4 to allow the pivoting at that level in a smoother way without twist. The arched elements 3 are seen straight in the central area of the haptic.

The haptics with arched J or C shape of the known Intraocular lenses normally have an open angle with regard to the optic, which would make difficult shooting with laser on the haptics even in its central half as would be hidden behind the iris, being needed a very wide pupil dilation or the employment of a 3 mirrors lens or other similar lens to make the shots with the laser.

For this reason, according to the present invention, the shape of the haptics is in double arch loops to maintain the characteristics and advantages of arch loops and at the same time to facilitate the visibility for the application of the laser on the elastic arched elements 3 present in the most central part in the haptics. The haptics have a central portion that make a narrow angle with regard to the optic of the lens 1 and closes more when the lens is introduced in the capsular sack due to the centripetal force exerted by the capsule. It is in this portion that the arched elements 3 are included, so they are more visible near to the center to be treated with the laser. The distal portion of the haptic can be more peripheral because it doesn't require to be visualized for the adjustment.

Other multiple variants of designs can use the principle object of this invention and therefore they constitute part of it, with haptics of diverse shapes in which the arched elastic components can be included with their curves placed in contrary anteroposterior directions with regard to this axis of the eyeball.

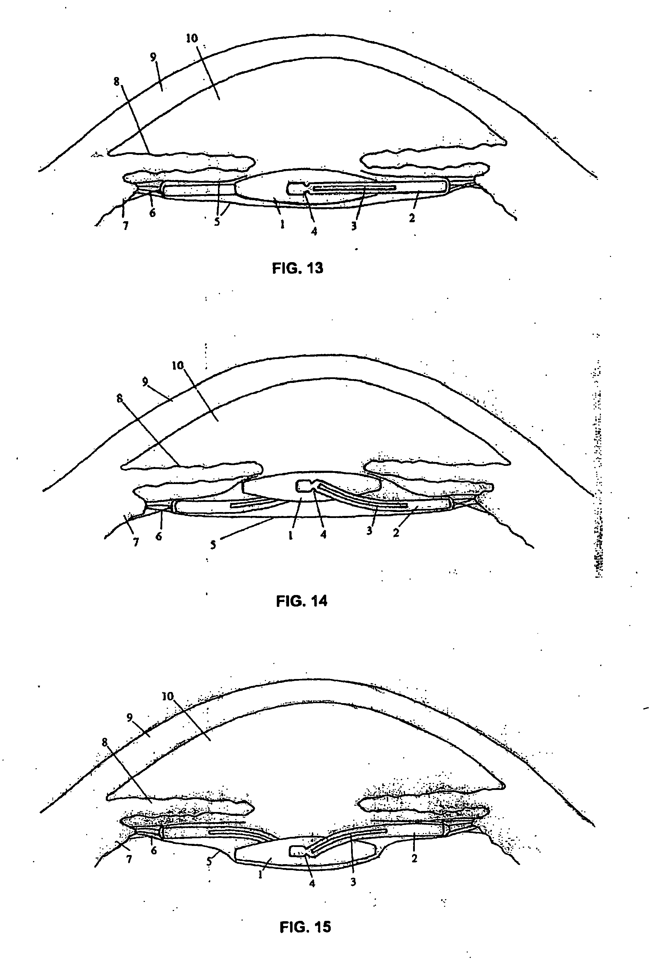

Next Figures 13, 14 and 15, illustrate how a lens according to the present invention can be implanted into a capsular sack 5.

Figure 13 shows the Intraocular Lens implanted in the capsular sack 5 and its relationship with the different anatomical structures of the anterior segment of the eye 10. The capsular sack 5 is fixed by its equator to the Ciliary Body 7 by the Zonules of Zinn 6.

Figure 14 shows the flexion of the arched elements 3 and the haptics, with the consequent displacement of the optic of the Intraocular Lens toward the iris 8 and the cornea 9. In this case the arched cylinders treated 3b in both haptics were the ones with the bend down.

Figure 15 shows the flexion of the arched elements 3 and the haptics, with the consequent displacement of the optic of the Intraocular Lens moving away from the iris 8 and the cornea 9. In this case the cylinders treated were 3a of both haptics which have the bend up.

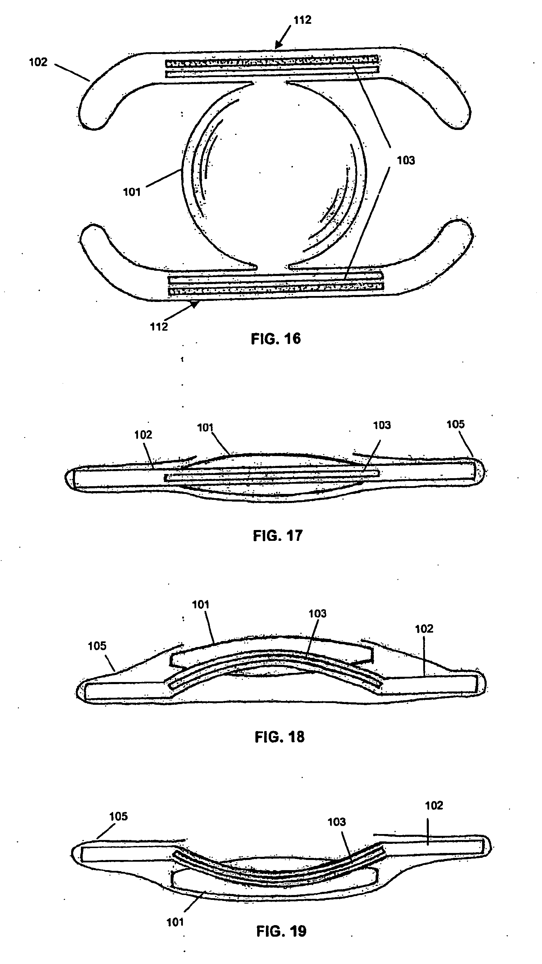

Figures 16 to 19 refers to a second embodiment of a lens according to the present invention.

Such second embodiment refers to a Intraocular Lens 101 with that principle or adjustable mechanism that can be used for implantation in the capsular bag 105 or in the Anterior Chamber of the eye. The lens 101 has two straight haptics or arms 102 symmetrical and parallel to each other, with the arched elements 103 in their center, where the bending takes place when being treated with laser. The haptics are joined to the optic 101 in their center and they are transverse to the optics and parallel among them.

In particular, Figure 17 shows the lens implanted inside the capsular sack 105. The Lens has not been treated with Laser, so the haptics maintain their straight shape. Contrary to the first embodiment, the Lens illustrated in this figure has the arched cylinders 103 placed exactly in the center of the haptics.

In Figure 18 the Lens has been treated with Laser weakening the cylinder bended up so that the cylinder acquire its original shape with its bend down bowing also the haptic 102 displacing it up in its central portion. The extremes of the haptics and arched elements are fixed, so the central flexion displaces the optic of the lens superiorly. Notice that with this design with the joint of the Optic to the haptic in the half portion of the haptic and the elastic arched elements, the shot to the elastic arched cylinder is done in the one with bend in opposite direction to the one that would be shot in the Lens of Figure 10 to achieve a displacement in the same direction.

In Figure 19 the Lens has been treated with Laser weakening the arch with bend down, so the arched element acquire its original shape with its bend up bowing also the haptic, displacing it down in its central portion, moving the optic 101 down.

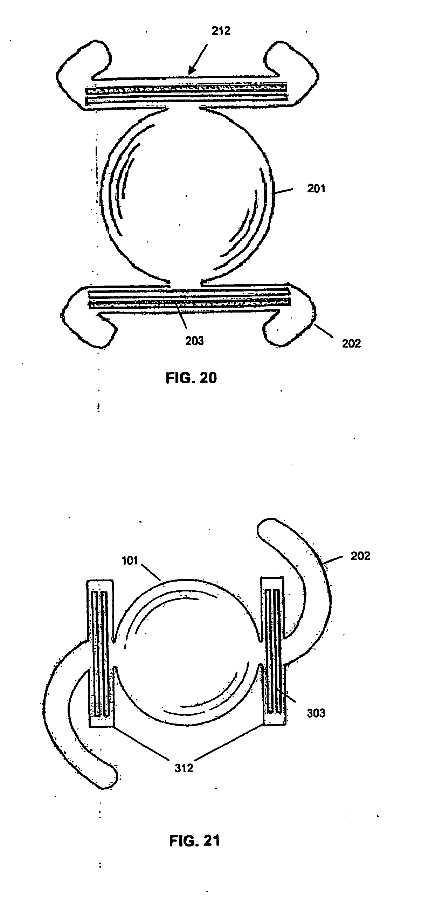

Figure 20 shows a third embodiment of a lens according to the present invention. A lens 201 differs from the previous one 101 for the shape of the four distal extremes of the haptics which, in the third embodiment are centrally curved or they can have a rounded, blunt end if for implantation in the capsular bag, or finer end if they are wanted for angular support in an Anterior Chamber Intraocular Lens. This design or with small modifications can be used in the Anterior or Posterior Chambers of the eye.

A fourth embodiment of a lens according to the present invention is shown in Figure 21. Such embodiment consists of a Lens with an optic area 301 and haptics with conventional "C" or "J" shape with a traverse bar 312 in the most central portion in the haptics, in which the arched elastic elements 303 are included which act adjusting the refraction by using the mechanism of arched cylinders as described in the previous models and object of this invention.

Other many designs could include the principle presented in this document including Intraocular lenses for iris fixation, for Anterior Chamber or Posterior chamber.

In the Intraocular Lens object of this invention the achieved effect can be reverted shooting with the Laser the arched element that is beside the one that was previously treated and that bends in opposite direction, in the same points that was shot the first arched component. This is very important in the case of overcorrection or if there is patient's dissatisfaction with the result of the adjustment. The refraction can be turned back to the existent previous to the treatment, or can be adjusted more finely.

This reversibility effect can give options to the Doctors, not available in the current state of the art. For example if an operated patient that reached a plano refraction in both eyes is not satisfied because does not have useful near vision; the Doctor can offer the patient to try monovision inducing a myopia of -1.0D or -1.50D in the non dominant eye to give near vision to the patient through this eye. This can be made in a non invasive way and if the patient is not happy with the new refraction, the lens is returned to the previous position or is moved back only partially to a refraction of -0.75D or another that is more comfortable for this particular patient.

The lens object of this invention would have special usefulness in the pediatric cataract surgery, because in the operated children the refraction changes during their development due to the growth of the eye. With this lens the refraction can be adjusted during the life time, maintaining the emmetropia throughout the diverse stages.

Adjustable haptics with the principle proposed in this invention can be used in phakic intraocular lenses to permit adjustment in the event of not good refractive results, and even perform refractive surgery to patients in earlier ages when the refractive defect has not still been stabilized.

## Claims

1. Intraocular Lens to be implanted in an eye of a patient, comprising an optic (1;101, 201, 301) and two or more haptics (2; 102; 202; 302) of foldable material, comprising a laser activated mechanism in the haptics that allows to adjust its position in the anterior-posterior axis of the eye by application of laser shots on the mechanism after the lens being implanted, in order to modify the patient's refraction by moving the optic away or bringing it near to a retina, so varying the existent refraction,
**characterized in that** the laser activated adjusting mechanism comprises one or more pairs of elastic arched elongated members (3; 103; 203; 303) located into respective fragments (12; 112; 212; 312) of each of said haptics (2; 102; 202; 302), the arched members (3; 103; 203; 303) of each pair being located one beside the other having opposite bends in anterior-posterior direction compensating their curves, so that in a rest untreated position the haptic is straight, while in a laser treated position the haptic assume a bend in the anterior-posterior direction, the laser treatment weakening the elastic strength of one of the elastic arched members (3; 103; 203; 303), so that the untreated one recovers its original curved shape bending the haptic and displacing the lens optic zone (1;101, 201, 301) in the anterior-posterior direction.

2. Intraocular Lens according to claim 1, wherein the elastic arched elements (3; 103; 203; 303) are made of an elastic biocompatible material, that can be weakened with laser and having a memory that allow them to recover their original arched shape after having changed it, each of said elastic arched elements (3; 103; 203; 303) being apt to bend the respective haptic fragment.

3. Intraocular Lens according to claim 1 or 2, wherein said arched elements can have a sectional shape as circle, bar, triangular, hexagonal or any other wanted and have, at least in one of their ends, a terminal shape so to avoid the arch to rotate and take a not wanted position inside the haptic.

4. Intraocular Lens according to anyone of the claims 1 to 3, wherein the elastic arched elements (3; 103; 203; 303) have marks (11), distributed along their length, indicating the points to shoot with a laser, so to obtain a predictable weakening of the material and a consequent bending of the haptic with a proportional displacement of the optic in the anterior-posterior axis of the eye.

5. Intraocular Lens according to anyone of the claims 1 to 4, wherein the lens is of a single piece of foldable material having two haptics (2) with double curve, each haptic having (2) a haptic fragment (12) housing the elastic arched members (3), the haptic fragment (12) forming a narrow angle with regard to the optic (1) of the lens, so that said elastic arched members (3) are accessible to the laser beam.

6. Intraocular Lens according to anyone of the claims 1 to 5, wherein each haptic (2) has a respective hinge portion (4) at the junction to the optic (1) to facilitate their movement

7. Intraocular Lens according to anyone of the claims 1 to 6, having two symmetrical and parallel straight haptics (101; 201), with the elastic arched members (103; 203) inserted in a central haptic fragment (112; 212) joined to the optic (101; 201).

8. Intraocular Lens according to claim 7, wherein the haptics ends are curved inward.

9. Intraocular Lens according to claim 7, wherein the haptics ends are curved outward.

10. Intraocular Lens according to anyone of the claims 1 to 9, having two haptics (302) having a "C" or "J" shape, each haptic (302) comprising an haptic fragment (312) housing the elastic arched members (303) transversally disposed with respect to the haptic and centrally joined to the optic (301).

11. Intraocular Lens according to anyone of the preceding claims, wherein the optic (1;101, 201, 301) can be a multifocal, aspheric, or toric optic.

12. Intraocular Lens according to anyone of the preceding claims, wherein the optic (1;101, 201, 301) comprises UV, blue light or other projection, alone or in combination therebetween.

13. Intraocular Lens according to anyone of the claims 1 to 12, wherein said elastic arched members (3; 103; 203; 303) have different colors depending on the direction of the bend in the anterior-posterior direction, to facilitate the recognition by the ophthalmologist of what element to shoot with laser to correct a myopic or hyperopic refractive defect.

14. Intraocular Lens according to anyone of the claims 1 to 13, wherein the arched elastic members of a pair are placed side by side.

15. Intraocular Lens according to anyone of the claims 1 to 13, wherein one of the arched elastic members of a pair is tubular and the other is placed therein.

## Patentansprüche

1. Intraokularlinse zur Implantation in ein Auge eines Patienten, welche eine Optik (1; 101, 201, 301) sowie zwei oder mehr Haptiken (2; 102; 202; 302) aus faltbarem Material aufweist, wobei die Intraokularlinse einen laseraktivierten Mechanismus in der Haptik aufweist, welcher die Einstellung ihrer Position in der Vorder-/Hinterachse des Auges durch Beaufschlagung des Mechanismus mit Laserstrahlen nach Implantation der Linse ermöglicht, um die Refraktion des Patienten durch Wegbewegen der Optik von oder durch Annäherung an eine Netzhaut zu modifizieren, wodurch die bestehende Refraktion verändert wird, **dadurch gekennzeichnet, dass** der laseraktivierte Einstellungsmechanismus ein oder mehrere Paare elastischer bogenförmiger länglicher Bauteile (3; 103; 203; 303) aufweist, welche in jeweiligen Fragmenten (12; 112; 212; 312) einer jeden Haptik (2; 102; 202; 302) angeordnet sind, wobei die gebogenen Bauteile (3; 103; 203; 303) eines jeden Paars nebeneinander angeordnet sind und entgegengesetzte Biegungen in die Vorder-/Hinter-Richtung aufweisen, welche ihre Krümmungen kompensieren, so dass die Haptik in Ruheposition oder unbehandelter Position gerade ist, wohingegen die Haptik in der laserbehandelten Position eine Biegung in die Vorder-/Hinter-Richtung annimmt, wobei die Laserbehandlung die elastische Festigkeit eines der elastischen bogenförmigen Bauteile (3; 103; 203; 303) schwächt, so dass das unbehandelte Bauteil seine ursprüngliche gekrümmte Form wiedererlangt, welche die Haptik krümmt und die Linsenoptikzone (1; 101, 201, 301) in die Vorder-/Hinter-Richtung verlagert.

2. Intraokularlinse nach Anspruch 1, **dadurch gekennzeichnet, dass** die elastischen bogenförmigen Elemente (3; 103; 203; 303) aus einem elastischen biokompatiblen Material hergestellt sind, welches durch einen Laser geschwächt werden kann, und ein Gedächtnis besitzen, welches es ihnen erlaubt, ihre ursprüngliche bogenförmige Form nach einer Veränderung dieser wiederzuerlangen, wobei jedes der elastischen bogenförmigen Elemente (3; 103; 202; 303) in der Lage ist, das entsprechende haptische Fragment zu krümmen.

3. Intraokularlinse nach Anspruch 1 oder 2, **dadurch gekennzeichnet, dass** die bogenförmigen Elemente die Querschnittsform eines Kreises, Stabs, Dreiecks, Sechsecks oder jede andere gewünschte Querschnittsform aufweisen können, und zumindest an einem ihrer Enden eine Endform aufweisen, so dass vermieden wird, dass sich der Bogen dreht und eine nicht gewünschte Position in der Haptik einnimmt.

4. Intraokularlinse nach einem der Ansprüche 1 bis 3, **dadurch gekennzeichnet, dass** die elastischen bogenförmigen Elemente (3; 103; 203; 303) Markierungen (11) aufweisen, welche entlang ihrer Länge verteilt sind, wobei die Markierungen die Laser-Bestrahlungsstellen anzeigen, um eine vorhersehbare Schwächung des Materials und eine resultierende Krümmung der Haptik mit einer proportionalen Verlagerung der Optik in die Vorder-/Hinterachse des Auges zu erzielen.

5. Intraokularlinse nach einem der Ansprüche 1 bis 4, **dadurch gekennzeichnet, dass** die Linse aus einem einzigen Stück faltbaren Materials hergestellt ist, welches zwei Haptiken (2) mit Doppelkurve aufweist, wobei jede Haptik (2) ein haptisches Fragment (12) aufweist, in welchem die elastischen bogenförmigen Bauteile (3) untergebracht sind, wobei das haptische Fragment (12) einen spitzen Winkel in Bezug auf die Optik (1) der Linse bildet, so dass die besagten elastischen bogenförmigen Bauteile (3) für den Laserstrahl zugänglich sind.

6. Intraokularlinse nach einem der Ansprüche 1 bis 5, **dadurch gekennzeichnet, dass** jede Haptik (2) einen jeweiligen Gelenkabschnitt (4) am Übergang zur Optik (1) aufweist, um deren Bewegung zu ermöglichen.

7. Intraokularlinse nach einem der Ansprüche 1 bis 6, welche zwei symmetrische und parallele gerade Haptiken (101; 201) aufweist, wobei die elastischen bogenförmigen Bauteile (103; 203) in ein zentrales haptisches Fragment (112; 212) eingeführt werden, welches mit der Optik (101; 201) verbunden ist.

8. Intraokularlinse nach Anspruch 7, **dadurch gekennzeichnet, dass** die Enden der Haptik nach innen gebogen sind.

9. Intraokularlinse nach Anspruch 7, **dadurch gekennzeichnet, dass** die Enden der Haptik nach außen gebogen sind.

10. Intraokularlinse nach einem der Ansprüche 1 bis 9, welche zwei Haptiken (302) mit einer "C"- oder "J"-Form aufweist, wobei jede Haptik (302) ein haptisches Fragment (312) aufweist, in welchem die elastischen bogenförmigen Bauteile (303) untergebracht sind, welche in Bezug auf die Haptik diagonal angeordnet sind und mittig mit der Optik (301) verbunden sind.

11. Intraokularlinse nach einem der vorhergehenden Ansprüche, **dadurch gekennzeichnet, dass** die Optik (1; 101, 201, 301) eine multifokale, asphärische oder torische Optik sein kann.

12. Intraokularlinse nach einem der vorhergehenden Ansprüche, **dadurch gekennzeichnet, dass** die Optik (1; 101, 201, 301) einen UV-, Blaulicht- oder anderen Schutz allein oder eine Kombination dieser aufweist.

13. Intraokularlinse nach einem der Ansprüche 1 bis 12, **dadurch gekennzeichnet, dass** die elastischen bogenförmigen Bauteile (3; 103, 203, 303) unterschiedliche Farben abhängig von der Richtung der Biegung in die Vorder-/Hinter-Richtung aufweisen, um die Erkennung für den Augenarzt zu erleichtern, welches Element mit dem Laser bestrahlt werden soll, um einen myopen oder hyperopen refraktiven Schaden zu korrigieren.

14. Intraokularlinse nach einem der Ansprüche 1 bis 13, **dadurch gekennzeichnet, dass** die bogenförmigen elastischen Bauteile eine Paars Seite an Seite angeordnet sind.

15. Intraokularlinse nach einem der Ansprüche 1 bis 13, **dadurch gekennzeichnet, dass** eines der bogenförmigen elastischen Bauteile eines Paars rohrförmig ist und das andere darin angeordnet wird.

## Revendications

1. Lentille intraoculaire à implanter dans un oeil d'un patient, comprenant une optique (1 ; 101, 201, 301) et deux haptiques ou plus (2 ; 102 ; 202 ; 302) réalisées avec un matériau pliable, comprenant un mécanisme activé par laser dans les haptiques qui permet d'ajuster sa position dans l'axe antérieur-postérieur de l'oeil, par l'application de tirs de laser sur le mécanisme après que la lentille a été implantée, afin de modifier la réfraction du patient en déplaçant l'optique à distance ou en l'amenant à proximité d'une rétine, modifiant ainsi la réfraction existante,
**caractérisée en ce que** le mécanisme d'ajustement activé par laser comprend une ou plusieurs paires d'éléments allongés arqués élastiques (3 ; 103 ; 203 ; 303) positionnés dans des fragments (12 ; 112 ; 212 ; 312) respectifs de chacune desdites haptiques (2 ; 102 ; 202 ; 302), les éléments arqués (3 ; 103 ; 203 ; 303) de chaque paire étant positionnés l'un à côté de l'autre, ayant des courbures opposées dans la direction antérieure-postérieure compensant leurs courbes, de sorte que dans une position non traitée au repos, l'haptique est droite, alors que dans la position traitée au laser, l'haptique adopte une courbure dans la direction antérieure-postérieure, le traitement au laser affaiblissant la résistance élastique de l'un des éléments arqués élastiques (3 ; 103 ; 203 ; 303), de sorte que l'élément non traité recouvre sa forme incurvée d'origine courbant l'haptique et déplaçant la zone optique de lentille (1 ; 101, 201, 301) dans la direction antérieure-postérieure.

2. Lentille intraoculaire selon la revendication 1, dans laquelle les éléments arqués élastiques (3 ; 103 ; 203 ; 303) sont réalisés avec un matériau biocompatible élastique qui peuvent être affaiblis avec le laser et ayant une mémoire qui leur permet de recouvrer leur forme arquée d'origine après en avoir changé, chacun desdits éléments arqués élastiques (3 ; 103 ; 203 ; 303) étant apte à courber le fragment haptique respectif.

3. Lentille intraoculaire selon la revendication 1 ou 2, dans laquelle lesdits éléments arqués peuvent avoir une forme transversale telle qu'un cercle, une barre, une forme triangulaire, hexagonale ou n'importe qu'elle autre forme voulue et avoir, au moins dans l'une de leurs extrémités, une forme terminale pour éviter la rotation de l'arc et qu'ils prennent une position indésirée à l'intérieur de l'haptique.

4. Lentille intraoculaire selon l'une quelconque des revendications 1 à 3, dans laquelle les éléments arqués élastiques (3 ; 103 ; 203 ; 303) ont des marques (11) réparties le long de leur longueur, indiquant les points à viser avec un laser, afin d'obtenir un affaiblissement prévisible du matériau et une courbure conséquente de l'haptique avec un déplacement proportionnel de l'optique dans l'axe antérieur-postérieur de l'oeil.

5. Lentille intraoculaire selon l'une quelconque des revendications 1 à 4, dans laquelle la lentille est réalisée d'un seul tenant avec un matériau pliable ayant deux haptiques (2) avec une double courbe, chaque haptique (2) ayant un fragment haptique (12) logeant les éléments arqués élastiques (3), le fragment haptique (12) formant un angle étroit par rapport à l'optique (1) de la lentille, de sorte que lesdits éléments arqués élastiques (3) sont accessibles au faisceau laser.

6. Lentille intraoculaire selon l'une quelconque des revendications 1 à 5, dans laquelle chaque haptique (2) a une partie d'articulation (4) respective à la jonction de l'optique (1) afin de faciliter leur mouvement.

7. Lentille intraoculaire selon l'une quelconque des revendications 1 à 6, ayant deux haptiques symétriques et parallèles droites (101 ; 201), avec les éléments arqués élastiques (103 ; 203) insérés dans un fragment haptique central (112 ; 212) assemblé à l'optique (101 ; 201).

8. Lentille intraoculaire selon la revendication 7, dans laquelle les extrémités de l'haptique sont incurvées vers l'intérieur.

9. Lentille intraoculaire selon la revendication 7, dans laquelle les extrémités de l'haptique sont incurvées vers l'extérieur.

10. Lentille intraoculaire selon l'une quelconque des revendications 1 à 9, ayant deux haptiques (302) ayant une forme de « C » ou « J », chaque haptique (302) comprenant un fragment d'haptique (312) logeant les éléments arqués élastiques (303) disposés de manière transversale par rapport à l'haptique et assemblés de manière centrale à l'optique (301).

11. Lentille intraoculaire selon l'une quelconque des revendications précédentes, dans laquelle l'optique (1 ; 101, 201, 301) peut être une optique multifocale, asphérique ou torique.

12. Lentille intraoculaire selon l'une quelconque des revendications précédentes, dans laquelle l'optique (1 ; 101, 201, 301) comprend des UV, de la lumière bleue ou une autre protection, seule ou en combinaison.

13. Lentille intraoculaire selon l'une quelconque des revendications 1 à 12, dans laquelle lesdits éléments arqués élastiques (3 ; 103 ; 203 ; 303) ont des couleurs différentes en fonction de la direction de la courbure dans la direction antérieure-postérieure, afin de faciliter la reconnaissance par l'ophtalmologiste de l'élément à viser avec le laser afin de corriger un défaut de myopie ou de réfraction hyperopique.

14. Lentille intraoculaire selon l'une quelconque des revendications 1 à 13, dans laquelle les éléments élastiques arqués d'une paire sont placés côte à côte.

15. Lentille intraoculaire selon l'une quelconque des revendications 1 à 13, dans laquelle l'un des éléments élastiques arqués d'une paire est tubulaire et l'autre est placé à l'intérieur de ce dernier.
